(19)

**Europäisches Patentamt**
**European Patent Office**
**Office européen des brevets**

(11) **EP 3 038 383 A1**

(12) **EUROPEAN PATENT APPLICATION**

(43) Date of publication:
**29.06.2016  Bulletin 2016/26**

(21) Application number: **15201019.5**

(22) Date of filing: **18.12.2015**

(51) Int Cl.:
*H04R 25/00* (2006.01)     *A61B 5/1171* (2016.01)
*G06K 9/00* (2006.01)     *G10L 25/78* (2013.01)
*G10L 15/25* (2013.01)     *H04R 3/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.12.2014  EP 14199978**

(71) Applicant: **Oticon A/S**
**2765 Smørum (DK)**

(72) Inventors:
- **GUO, Meng**
  **DK-2765 Smørum (DK)**
- **PEDERSEN, Michael Syskind**
  **DK-2765 Smørum (DK)**
- **JENSEN, Niels Søgård**
  **DK-2765 Smørum (DK)**
- **JENSEN, Jesper**
  **DK-2765 Smørum (DK)**

- **MINNAAR, Pauli**
  **DK-2765 Smørum (DK)**
- **BJELOSEVIC, Adis**
  **DK-2765 Smørum (DK)**
- **LAPLANTE-LÉVESQUE, Ariane**
  **DK-2765 Smørum (DK)**
- **OLSEN, Ole Fogh**
  **DK-2765 Smørum (DK)**
- **WESTGÅRD, Bo**
  **DK-2765 Smørum (DK)**
- **BRAMSLØW, Lars**
  **DK-2765 Smørum (DK)**
- **NIELSEN, Claus**
  **DK-2765 Smørum (DK)**
- **RØNNE, Filip Marchman**
  **DK-2765 Smørum (DK)**

(74) Representative: **Mikkelsen, Jacob**
**Oticon A/S**
**WDH Group IP**
**Kongebakken 9**
**2765 Smørum (DK)**

(54) **HEARING DEVICE WITH IMAGE CAPTURE CAPABILITIES**

(57)     The present disclosure relates to hearing devices including an image capture device. Features of sound processing may be based on analysis of images from the image capture device.

Fig. 1

EP 3 038 383 A1

1                 EP 3 038 383 A1                 2

## Description

The present disclosure relates to hearing devices having an image capture device. The captured images may be still images, a sequence of images and/or video.

Hearing devices usually are intended to present to a user a target signal which is better audible and/or intelligible and/or pleasant, etc. than what the user experiences without his/her hearing device. Sound processing in environments having multiple sound sources is cumbersome and detection and separation of active sound sources is an active field when using instruments having a directional input system. Therefore, in one sense, one purpose of a hearing device is to filter incoming acoustic signals and present to the user the relevant part of the acoustic scene while suppressing the irrelevant part, e.g. background noise.

Hence, an improved apparatus would be advantageous. It is an object of the present disclosure to provide an alternative to the prior art.

In particular, it may be seen as an object of the present disclosure to provide a hearing device that solves, or at least alleviates or at least provides an alternative solution to the above-mentioned problems.

Thus, the above-described object and several other objects are intended to be obtained in a first aspect by providing a hearing device to be worn by a user. The hearing device may be provided with a housing configured for being worn by the user at an ear of the user. The housing may be adapted for being mounted at or behind a pinna, i.e. behind the ear, in the ear canal, either fully within the canal or partly in the ear canal. Within the hearing device industry these configurations are commonly referred to as e.g. In-The-Ear, ITE-full shell, ITE Half-shell, In-The-Canal, Mini-canal, Completely-In-the-Canal, etc. The hearing device may further comprise an input transducer for receiving sound signal. The input transducer may be included in the housing, or be an external unit in communication with the housing, or a combination of input devices constituting the input transducer, e.g. one input device located in a housing and one input device located externally to the housing, where each may be a combination of two or more input devices. Further, the hearing device may receive sound signals from an external device remote from the hearing device. This could for instance be a device worn in a string around the neck of the user or from a device worn by anther person.

The hearing device may further comprise a processor for processing the received sound signal. This processor may process received sound so as to compensate for a user's specific hearing loss, which may include gain correction, frequency transposition, compression, expansion, or the like. The instructions for performing the processing may be stored in a memory of the hearing device associated with the processor. Additional sound information may be stored in the processor and may be added to the signal before being presented to the user. This may include natural or generated sound. The additional sound information may be selectively added e.g. based on the type of acoustic environment determined based on an image capture device as described later and/or the input device.

The hearing device may further comprise an output transducer for providing the processed sound signal perceivable as sound to the user. This output transducer may e.g. be a speaker unit, often referred to as a receiver or a device providing electrical stimulation of the cochlear, or a vibrational unit providing vibrations to the inner ear via bone-born vibrations or a combination of several units.

The hearing device may further comprise an image capture device in communication with the processor. The processor may be configured to detect presence of a face via the image capture device. The processor may be configured to determine time instants of voice presence and voice absence from the face. The processor may be adapted to operate (sound) signal processing algorithms based on the detection. The image capture device may be sensitive to visual light, infrared radiation, ultraviolet radiation, or any other suitable radiation. Different kinds of image processing are possible, as will be apparent from the present disclosure. The image capture device may serve at least the purpose of target and/or noise source localization and/or presence, i.e. to provide information about the location and/or presence of target and/or potentially noise sources relative to the hearing device user. This may include detecting that a person or face, or multiple persons or faces, is/are present in the image capture device's field of view. Further, the image capture device may serve at least the purpose of providing face and/or mouth movement information, i.e. to generally provide information about the target speaker or speakers, and potentially of interfering speakers, and, in particular, information related to mouth/lip movements, e.g. mouth area/shape, lip positions, etc. These two purposes may be combined to even further enhance sound processing. The image capture device may also serve to determine the acoustic environment either on its own or in combination with information from the input device, e.g. if only one person is determined to be present in an image and the sound pressure level from the input device is below a certain threshold, it may be established that the environment can be classified as quiet and the sound processor may process sound accordingly, e.g. more aggressive noise suppression for improved signal-to-noise ratio.

In one aspect a hearing device may have a first part configured to be positioned behind the pinna of a user and a second part configured to be positioned in the ear canal of the user, and a third part configured to mechanically connect the first part to the second part. The first part could comprise an input transducer for receiving sound signal, a processor for processing the received sound signal, and an image capture device in communi-

2

cation with the processor. The processor could be any suitable general-purpose processor or a specific signal processor. The image capture device could be positioned in the housing so that the image capture captures images in the direction of the nose of the wearer; this could also be referred to as the looking direction. Further, the processor could be configured to detect presence of a face via the image capture device, and determine time instants of voice presence and voice absence from the face, and the processor could be adapted to operate signal processing algorithms based on the detection. Still further, the hearing device could include an output transducer for providing the processed sound signal perceivable as sound to the user. The output transducer could be configured to be positioned in either the first part or the second part. In case the output transducer is positioned in the first part, the third part may be a tube for airborn transmission of sound from the output transducer. In case the output transducer is positioned in the second part, the third part may include at least two electric wires for transmission of signals representing processed sound to the output transducer. The output transducer should transform the electrical signal to a signal that the user could perceive as sound.

Examining an image, or sequence of images, a mouth/lip region may be identified where after movement may be detected. The identification or classification of a region as 'mouth' may be preceded by a step of identifying a face or head.

In general, if location information on target and/or noise sources is available, several signal processing steps in a hearing device may be improved over a hearing device not having this information. For example,

- a1) the spatial response (beam pattern) of any beam forming algorithm may be steered in the direction of the identified target source,
- a2) spatial nulls of the beam pattern may be steered in the direction of identified noise sources.
- a3) knowing the, relative, direction to the target source allows emphasizing spatial cues of the target source.

In general, if face/mouth/lip movement information is available regarding an identified target speaker, this information may be used to improve the voice-processing algorithms on-board the hearing device. For example,

- b1) mouth movement information may be used for target voice activity detection, which directs the processing of noise reduction algorithms on-board the hearing device.
- b2) more detailed features of the mouth/lip movement of the target speaker, e.g. open mouth area, mouth shape, etc., may help inform a hearing-loss compensation system to improve its performance, e.g. in the presence of speech transients.

- b3) detailed information about target mouth/lip movements provide additional information (over the microphone signals) which can improve the performance of the noise reduction algorithms on-board the hearing device.

The image capture device may be arranged so that when the hearing device is worn, the image capture device captures images in the looking direction of the user. As the hearing device will be attached or mounted to the users head, the looking direction relative to the hearing device will most likely not change substantially during use. By looking direction is meant the direction that the face of is user is facing, could be seen as the direction of the nose.

The image capture device may capture single images, sequences of images or continuously capture images or video. The capture may be performed according to a predefined schedule, e.g. periodically with fixed or variable time interval. For reducing power consumption, the schedule may be adapted to capture less number of images in quite situations or stabile situations with few changes. The capture may be performed or initiated subsequent to a specific sound event being detected using the input transducer, this could be in a situation where more than one sound source is detected. The capture may be performed continuously, e.g. when started by the user via an interface or from the moment where the hearing device is turn on.

The image capture device may be positioned in a housing of the hearing device, and the hearing device is configured to be positioned at an ear of the user. This allows detection of e.g. movement of the head and/or capturing images from a well-defined direction relative to the head of the user. The image capture device may alternatively be positioned remote from a housing of the hearing device and the image capture device may then be in wired or wireless communication with the processor. Memory devices may be included for buffering and/or storing images. A remote location may be needed if images are to be captured in the direction of the face of the user and the hearing device is positioned at least partly in the ear canal of the user. This may e.g. be achieved via an image capture device mounted in or on a set of glasses or in a body-worn housing, e.g. for being worn at the chest of the user or a device located on the pinna and either in wired or wireless communication with the in-ear part. Multiple image capture devices may be combined, e.g. to ensure a more complete field of view. Also, image capture devices facing different directions. As an example of multiple image capture device may be one or more looking forward and one or more looking backwards.

The image capture device may be in communication with a dedicated image processor or may utilize the processor also used for sound processing. In the present text, only processor is referred to, but both options are meant to be covered.

The processor may be configured to detect lip movement in image sequences captured by the image capture device. This allows the processing of the sound signal to be optimised according to the detected lip movements, e.g. by adapting noise suppression, gain, speech enhancement and/or other suitable processing. The processor may further be adapted to analyse the sound signal to detect speech envelope and correlate the speech envelope to detected lip movement to identify direction to an active speech source relative to the image capture device. In case multiple speech sources are present, selecting the currently active speech source is made less cumbersome if detection was based on sound analysis alone, e.g. in noisy environments with multiple potential sound sources.

The input transducer may include a directional microphone system having adaptable directionality, and the processor may be configured to adapt the directionality of the directional microphone system. This adaptation may be performed based, at least partly or in full, on images captured by the image-capturing device. Other inputs for adapting the directionality may include instructions from the user, e.g. that a certain directionality is preferred, e.g. back-wards of the user, further up or down relative to eye level, left-right priority, e.g. when the user is sitting in a car, or any other reason.

A hearing device with a perfect directionality system would enable its end-user to focus on the sound source of interest, while suppressing the sounds that are not of interest. Adding an image device such as a 3D camera to the hearing device would provide real-time 3D imaging information about the environment. The hearing device input transducer already receive acoustical waves. However, acoustical waves cannot accurately identify the location of the sound source in all situations. The accuracy of the microphones varies based on the acoustical environment (e.g. reverberation, background noise). A 3D camera receives electromagnetic waves, both visible light and IR light, which then could be processed and turned into positioning information about the surroundings.

The combination of the information arising from the acoustical waves and the electromagnetic waves may be used by the beamformer of the directionality system. The electromagnetic waves could act as a supplement to the acoustical waves when detecting a position of an object. Furthermore, the directionality system, with the beamforming, would adapt more precisely in unpredictable, fluctuating acoustical environments.

The hearing device may include a communication device for communicating with a second hearing device positioned at an opposite ear of the user. The second hearing device may also include an image capture device, wherein the processor is configured to determine angle to an object recorded by both image capture devices and the processor is further configured to adapt directionality of the directional microphone system accordingly. The two hearing devices may be of the same type, including basically the same features. Alternatively, two hearing device may be of different types, e.g. one hearing device may for instance be a so-called Behind-The-Ear-type hearing device, and the other hearing device may be of an In-The-Ear-type. Using two hearing devices positioned at a distance between them, i.e. one at each ear, each hearing device having an image capture device, allows for advanced image processing, which could include detecting distance to objects in the image. This could be determining distance to a detected talker, whereby adaptation of the input transducer may be performed so as to enhance speech from that source.

In a second aspect, the present disclosure presents a method of operating a hearing device including an input transducer for receiving sound signal, a processor for processing the received sound signal, an output transducer for providing the processed sound signal perceivable as sound to the user, and an image capture device in communication with the processor. The hearing device may be of the type discussed in relation to the first aspect and may include any or all features discussed in that relation. The image capture device may be arranged so that when the hearing device is worn the image capture device captures images in the looking direction of the user. The method may comprise the image capture device capturing one image or a sequence of images. The method may comprise the processor detecting presence of a face via the image capture device and determining time instants of voice presence and voice absence from the face. The time instants may be an indication of the two states, presence vs absence. The method may comprise the processor adapting the processing of the sound signal accordingly. A sequence of images may be a number of images captured in temporal sequence. A multitude of images may be a number of images taken at different points in time, where the time difference between the images are not necessarily equal.

The input transducer may include a directional microphone system having adaptable directionality, and the method may further comprise a step of detecting a face in the image or sequence of images and adapting the directionality accordingly. This adaptation of the directionality may include noise suppression, speech enhancement or any other suitable adaptation and/or processing.

In the event that multiple faces are detected in the captured image or sequence of images, the method may include detecting lip movements of the detected faces, and the processor is then adapted to analyse the sound signal to detect speech envelope and correlate the speech envelope to detected lip movement to identify direction to an active speech source relative to the image capture device.

When a second hearing device including an image capture device is positioned at an opposite ear of the user, the method may include correlating images or image sequences from the two image capture devices, or at least information extracted based on the captured images, to determine distance to an object present in both the images or image sequences. The object may be an obstacle, such as wall, person, kerb, lamppost, etc. Two hearing devices, or at least the image capture device parts thereof, may be positioned at a distance between them, e.g. one at each ear, but, as mentioned, other positions are possible e.g. body-worn or the frame of a set of glasses. This allows for enhanced detection possibilities based on image analysis, e.g. determining distance to an object in the images, as the distance to the object will be reflected by the relation of the position of the object in each image and the distance between the two image capture devices.

The processor may analyse images from the image capture device to detect vertical and/or horizontal movement of the head of the user, i.e. inclination and/or yaw. This may be used for adapting e.g. directionality of a directional microphone system, and/or for detecting changes in head orientation of the user, e.g. tilt and/or turn. The adaptation of the directionality may be performed to maintain the directionality towards a desired target.

Analysing image or image sequence may include recognising and identifying a person as a talker known to the hearing device via a database of known talkers. One or more characteristics of a person may be stored and used for identifying the person using the image capture device. This may include eye and/or nose and/or mouth position or relative the each of the corresponding other or other suitable visual characteristic used for face detection. When a person is then recognised, the hearing device processing may be adapted according to the recognized talker, e.g. if the person is a spouse certain processing may be desired, while other members of the users family may require other types of processing, e.g. speech enhancement specifically aimed at enhancing speech from children.

The image capture device may be used for detecting objects other than people. The image capture device may be used for detecting that an object is near the wearer. This could be useful for a warning system for the user, e.g. to be able to receive warnings or notification that the user is nearing a stationary object, such as a lamppost or wall, or for instance a curb or movable objects such as other people or cars or the like. This will particular be useful if the user in addition to a hearing loss has a visual impairment. Combining two such assistive devices into one device is a benefit to the user who will feel more comfortable navigating the streets with a combined hearing aid and vision aid combined into one device or set of devices positioned at one or two ears.

Further, the hearing device may include a vibrational device providing vibrational feedback to the user. This vibration signal may then be used as a warning signal to the user, e.g. the amplitude and/or frequency of the signal could provide the user with information on the distance to an object. This could be a weak signal indicating that the distance is above a certain threshold and a stronger signal indicating that the distance is shorter than the threshold. The vibration intensity and/or frequency may follow a scale, possible including one or more thresholds or a scale directly proportional to the distance. In a binaural system it would be possible to indicate to the wearer the direction to the object, e.g. by applying the vibration in only one side, or stronger in one side etc. If the object or obstacle appears to the right, the right hearing aid should vibrate to indicate to the wearer to slightly turn left, and vice versa. If the object is right in front of the wearer, both hearing aids could vibrate synchronously. If the wearer is approaching objects on both sides, the hearing aids could vibrate asynchronously.

The vibrational feedback could include a manual override for situations where the user does not want this feedback. In addition, in many countries, audible signals are already part of the infrastructure to support blind citizens in navigating outdoors, e.g. at pedestrian crossings.

Quite simply, one purpose of this tactile navigation feature is to prevent the visually and hearing impaired wearer from bumping into things or people while moving about while at the same time keeping his or her hands free.

The hearing device could also include a GPS device, which provides information to the user about which way to make a turn, and how far away you are from target, etc. This feature could also be used for location-specific settings of programs etc. This could include a switching to a special program when the user is outside or when the user enters a special location.

In a third aspect, the present disclosure presents a computer program product configured to execute the steps of the method according to the second aspect. This computer program product may be configured to be executed by a processor in a hearing device, e.g. a hearing device as discussed in relation to the first aspect.

In general, the embodiments of the present disclosure preferably seeks to mitigate, alleviate or eliminate one or more of the above-mentioned disadvantages singly or in any combination.

The individual features of each aspect may each be combined with any or all features of the other aspects. These and other aspects, features and/or advantages will be apparent from and elucidated with reference to the embodiments described hereinafter.

BRIEF DESCRIPTION OF THE FIGURES

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

Fig. 1 schematically illustrates a hearing device,
Fig. 2 schematically illustrates a hearing device mounted at an ear,
Fig. 3 schematically illustrates a hearing device mounted at an ear of a user, seen from above,
Fig. 4 schematically illustrates a binaural hearing system mounted at two ears of a user, seen from above,
Fig. 5 schematically illustrates steps of a method,
Fig. 6 schematically illustrates correlating lip movements to speech envelope of a signal, and
Fig. 7 is a schematic illustration of a hearing device .

DETAILED DESCRIPTION OF AN EMBODIMENT

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A hearing device may be a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading airborne acoustic signals into the ear canal or with a receiver/ loudspeaker arranged close to or in the ear canal such as in a Behind-the-Ear type hearing device, and/ or ii) arranging the hearing device entirely or partly in the pinna and/ or in the ear canal of the user such as in a In-the-Ear type hearing device or In-the-Canal/ Completely-in-Canal type hearing device, or iii) arranging a unit of the hearing device attached to a fixture implanted into the skull bone such as in Bone Anchored Hearing device or Cochlear Implant, or iv) arranging a unit of the hearing device as an entirely or partly implanted unit such as in Bone Anchored Hearing device or Cochlear Implant. A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a SmartPhone or other electronic device, the SmartPhone/ electronic device possibly executing an application that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear Implant.

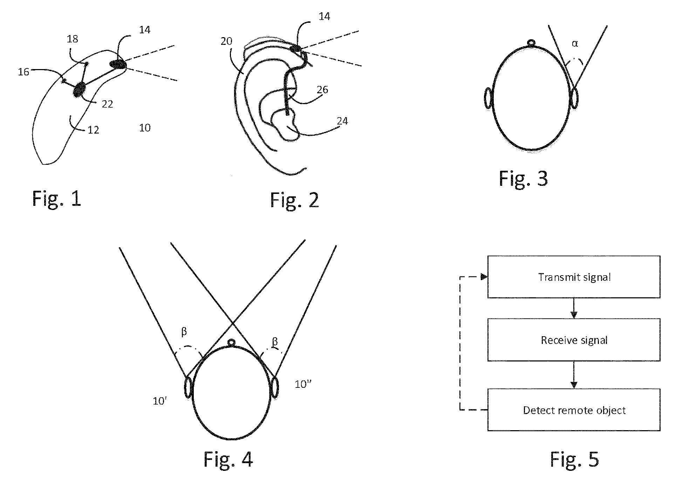

Fig. 1 schematically illustrates a hearing device 10 to be worn by a user. This hearing device 10 is provided in a housing 12 configured for being worn at, or behind, an ear 20 of the user, as illustrated in Fig. 2. Within the hearing device industry, hearing device configurations are commonly referred to as e.g. Behind-The-Ear, In-The-Ear, ITE-full shell, ITE Half-shell, In-The-Canal, Mini-canal, Completely-In-the-Canal, etc. The configuration illustrated in Fig. 1 is a so-called Behind-The-Ear, whereas the configuration in Fig. 2 is a so-called Receiver-In-the-Ear. Alternatively, the hearing aid as shown in Fig. 2 could be composed of a behind-the-ear element holding an image capture device and the main components of the hearing compensation electronics could be housed in the in-the-ear part. This would allow the image capture device and associated properties to be an optional element, which could be selectively coupled to the in-the-ear hearing aid. Other distributions of electronic components are of cause also possible. The connection for transmission of data from/to the image capture device, i.e. between the behind-the-ear part and the in-the-ear part, could be wire-based as shown in Fig. 2, but could alternatively be wireless.

In Fig. 2, the connector 26 is a sound tube configured for acoustically transferring an acoustical signal to the user's ear canal. Alternatively, the connector comprises an electrically conductive wire or wires transferring an electrical signal to a speaker or receiver unit placed at the user's ear canal entrance. Further alternatively, an electrically conductive wire or wires may be used for transferring an electrical signal to the cochlear of the user, or even further alternatively a vibrational element transferring vibrational signals to the skull of the user. Other types of devices may be used for conveying a signal perceivable as sound to the user.

Currently, hearing devices aim at filtering incoming acoustic signal and present to the hearing device user the relevant part of the acoustic scene while suppressing the irrelevant part based solely on microphone signals, i.e. acoustic information. For example, based on the microphone signals, the hearing device tries to detect when a target signal is present, i.e. speech activity detection, the direction of the target relative to the hearing device user, the direction to point-source interferers, etc. Based on acoustic information alone, this task is difficult and often error prone.

In Figs. 1 and 2 the housing 12 is adapted for being positioned at or behind a pinna 20, i.e. behind the ear of the user where it is held in place between the head and the pinna. In this position, an image capture device 14 is able to capture images in the direction of the field of view of the user as illustrated e.g. in Fig. 3. In Fig. 1 and 2 the vertical part of the field of view is illustrated by the punctured lines emanating from the image capture device 14. This looking direction follow the direction of the nose of the user, and not the eyes.

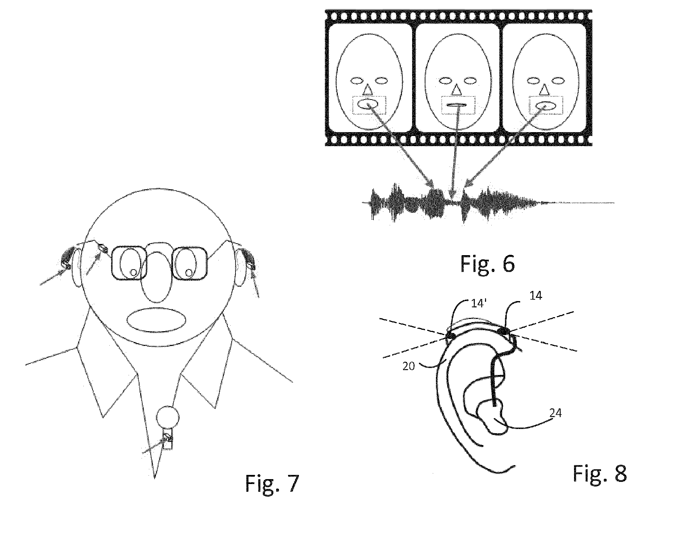

The hearing device 10 further comprises an input transducer for receiving sound signal. The input transducer is included in the housing 12, but not illustrated here. Alternatively, the input transducer may be an external unit in communication with the hearing device. Such an external input transducer may for instance be worn at the chest of the user, or even on a different person, e.g. a spouse or the like, as illustrated in Fig. 7.

The hearing device 10 further comprises a processor 22 for processing the received sound signal. This processor 22 processes the received sound to compensate for the users specific hearing loss, which may include gain correction, frequency transposition, compression, expansion, or the like. The user has usually previously visited a hearing care professional to have his or her hearing measured to determine the hearing loss, or in some other way have provided information regarding the hearing capability of the user to the hearing device so that suitable compensation may be performed to sound. Further, the processor is configured to process other data, as will be explained later.

The hearing device 10 further comprise an output transducer, in Fig. 2 illustrated as the in-ear part 24, for providing the processed sound signal perceivable as sound to the user. This output transducer 24 is here a speaker unit providing an acoustic output, and often referred to as a receiver within the hearing device industry, but may, in other instances, be a device providing electrical stimulation directly to the cochlear, or a vibrational unit providing vibrations to the inner ear via bone-born vibrations. The connector 26 connects the in-ear part 24 and the housing 12. Electrical wires make electrical connections between the receiver in the in-ear part 24 and the electronics in the housing 12.

As mentioned, the hearing device 10 further comprises an image capture device 14 in communication with the processor 22. The processor 22 is configured to adapt the processing of the sound signal based on input from the image capture device 14. Different kind of processing adaptions are possible.

The image capture device 14 is capable of capturing single images, sequences of images or continuously capture images or video. The capturing is configurable to be performed according to a predefined schedule, e.g. periodically with fixed or variable time interval. The image capture may be performed continuously, e.g. when started by the user via user interface or simply from the time the hearing device is turn on. The choice of scheduling may be left to the user, e.g. to be defined via a user interface.

For reduced power consumption, the schedule may be adapted to capture a lesser number of images in certain situations. This could be adapted autonomously by the hearing device 10 e.g. in periods where the sound environment is quite or have low noise, or stabile situations where only few changes in the environment are detected, especially changes in the sound environment.

The capture of images may be performed or initiated subsequent a specific sound event detected using the input transducer. This could for instance be a situation where more than one sound source is detected using audio analysis, e.g. two competing speakers.

When worn at the ear of the user, the image capture device further allows detection of movement of the head of the user, including tilting forward, sideways, turning or yaw. As the hearing device 10 includes an input transducer in the form of a directional microphone system 16, 18 with adaptable directionality, it is possible to adapt the pickup direction of the directional microphone system in response to detecting movement of the head. The width of the directional microphone system have certain width, which limits the need for accuracy of the pickup direction towards the sound source. The width of the directional microphone system may be adapted depending on several factors, including stability of direction to the source, noise level in the surroundings, sound level from the source or a combination of several factors.

Further, the image capture device allows the hearing device to compensate for placement of the hearing device in a position other than what is expected by the instrument, e.g. further back on the pinna or more askew on the pinna than expected.

Detection of movement of the head is useful in situations where an active speaking source is present, e.g. in front of the user, and the user moves his or her head, then the directional microphone system may be adapted so that the user is still receiving sound from the active speaker.

Such situations could be a user being at a dinner party and having a conversation with a person sitting across from the user. At one point in time, the user has his or her face directed directly at the speaker and the directional microphone system is directed forward. The user then moves his or her head towards the table to eat, but the speaker is still speaking, therefore, detection of movement of the head via the image capture device allows the directional microphone system to be adapted so as to keep picking up the sound from the speaker even though the face of the user is facing the table. The degree and speed of movement may be extracted from the images and used in adaptation of the directional microphone system.

As illustrated in Fig. 7 the image capture device may be remote from the housing of the hearing device 10. Fig. 7 schematically illustrates a number of possible locations of an image capture device. The image capture device located at the chest of the user and the image capture device at the temple of the glasses are here in wireless communication with the processor, but may alternatively be in wired communication with the processor. These locations could be useful if images are to be captured in the direction of the face of the user and the hearing device itself is positioned at least partly in the ear canal of the user.

Such a remote image capture device may be mounted in or on a set of glasses or in a body-worn housing, e.g. for being worn at the chest of the user, as illustrated in Fig. 7.

The processor comprises image analysis software for detecting lip movement in image sequences captured by the image capture device. One way of achieving this is illustrated in Fig. 6. The detection of lip and/or mouth movement allows the processing of the sound signal to be optimized according to the detected movements. Lip movement is usually associated with speech, and the optimization include adapting noise suppression, gain, speech enhancement and/or other suitable processing.

The processor is also be adapted to analyze the sound signal to detect speech envelope and correlate the speech envelope to detected lip movement to identify direction to an active speech source relative to the image capture device. If the user is attending a cocktail party, multiple speech sources will be present and selecting one of the multiple active sources can be difficult using only audio processing. By correlating the speech envelope and lip movement, as outlined in Fig. 6, the selection of currently active speech source is made less cumbersome than if detection was based on sound analysis alone.

As mentioned, the input transducer includes a directional microphone system having adaptable directionality, and the processor is able to adapt the directionality of the directional microphone system. This adaptation may be based, at least partly or in full, on images captured by the image-capturing device. Other inputs for adapting the directionality may include instructions from the user, e.g. that a certain directionality is preferred, e.g. back-wards of the user, further up or down relative to eye level, left-right priority, e.g. when the user is sitting in a car or any other reason. Using the user interface as described in relation to Fig. 1, the user may be presented with a graphical view to change the preferred directionality. The hearing device may also include one or more accelerometers. This one or more accelerometers may then be used to determine inclination of the hearing device

Fig. 4 illustrates a hearing system including two hearing devices positioned at opposite sides of the head. The hearing devices 10' and 10" each includes a communication device for communicating with a similar hearing device positioned at an opposite ear of the user.

The second hearing device 10" also includes an image capture device, and at least one of the processors is configured to determine the angle to the object, the processor adapts directionality of the directional microphone system accordingly.

The two hearing devices are in this example identical, i.e. of the same type including, basically, the same features, but alternatively, one hearing device may for instance be a so-called Behind-The-Ear-type hearing device, and the other hearing device may be of a different type so that the two hearing devices are not of identical types.

Using two hearing devices positioned at a distance between them, e.g. one at each ear, and each instrument having an image capture device, allows advanced image processing, e.g. estimating distance to persons in the image. This could be determining distance to a detected talker, whereby adaptation of the input transducer is performed to enhance speech from that specific source.

With face detection techniques, the time varying area of the mouth is found and correlated with the speech envelope the system is able to detect who is talking, or at least in which direction relative to the hearing device the speech is originating. Further, the direction of the talker can be used to suppress noise from other directions. The time varying area of the mouth may also be used to create a gain envelope which may be used for noise suppression/ speech enhancement. Envelopes may be created for all simultaneous talkers.

Camera on the hearing devices in combination with face recognition may be used to recognize a specific talker and the hearing device processing may be adapted according to the recognized talker.
The cameras may be used as an alternative communication channel. Data such as audio may be encoded into a picture/movie, which is recorded by the camera and decoded into an audio stream and presented to the hearing device user.

Fig. 4 schematically illustrates a top-down view of a user's head where two hearing devices are mounted at respective ears of the user. An image capture device is included in each of the hearing devices. The image capture device of each instrument is arranged so they capture images within the angle β. The field of view of the image capture devices overlaps so that an object in the combined field of view is visible in both images. This allows distance to the object to be estimated, e.g. via triangulation or the like method, along with an estimate of the distance between the two hearing devices.

Fig. 5 schematically illustrates steps of a method of operating a hearing device. The hearing device to be operated by this method is of a type including an input transducer for receiving sound signal, a processor for processing the received sound signal, an output transducer for providing the processed sound signal perceivable as sound to the user, and an image capture device in communication with the processor.

The hearing device may advantageously be of the type discussed above and may include any or all features discussed in that relation. When performing the method, the image capture device should be arranged so that the image capture device captures images in the looking direction of the user. The method comprises the image capture device capturing one image or a sequence of images.

The method may comprise the processor adapting the processing of the sound signal based on the captured one image or sequence of images.

When using a hearing device where the input transducer includes a directional microphone system having adaptable directionality, the method can be performed with a step of detecting a face in the image or sequence of images and adapting the directionality accordingly. This adaptation of the directionality may include noise suppression, speech enhancement or any other suitable adaptation.

In the event that multiple faces are detected in the image or sequence of images, the method includes detecting lip movements of the detected faces, and the processor analyses the sound signal to detect speech envelope and correlate the speech envelope to detected lip movement to identify direction to an active speech source relative to the image capture device.

Fig. 6 schematically illustrates correlating lip movements to speech envelope of a signal.

The following is a more detailed description of a hearing device noise reduction system, which makes use of signal(s) of one or more input devices, but which uses additional information regarding the target source, here a video stream of mouth movement information.

For the sake of this example, let $X(t)$ denote a noisy microphone signal (as a function of time t). Alternatively, X(t) could be the output of a noise reduction algorithm on the hearing device. Furthermore, let $V(t)$ denote a (potentially normalized) signal which describes the target mouth movement as a function of time; for example, V(t) could be the open-mouth area (normalized to the range [0;1]) as a function of time.
A simple, heuristic method to use the additional information carried in $V(t)$ to improve the quality of $X(t)$ would be to form the signal $S(t) = X(t)*V(t)$, where * denotes pointwise multiplication. This means that when $V(t)$ is "small", speech activity is absent, and the noise in $X(t)$ is suppressed, while when $V(t)$ is "large", speech activity is present, and $X(t)$ is not attenuated. Other such methods may be devised for processing $X(t)$ based on $V(t)$.

In the case where the image capture device is not part of the hearing device housing, but part of e.g. a pair of glasses or other external device, the whole image is not necessarily transmitted to the hearing device. Instead, the information transmitted to the hearing device could be area of mouth, identified person or other meta-information extracted from the recorded image or images.

Noise reduction or speech enhancement can be approached as the problem of estimating the underlying clean speech signal, given some observable information, e.g. noisy microphone signals, a mouth video stream, etc. Below is described a statistically optimal approach to solve this speech enhancement problem.

Let S denote the quantity of interest. In the current context, S could e.g. be the underlying clean target signal, a time frame of the target signal, a complex-valued DFT coefficient of a given time frame of the target signal, etc.

Similarly, let $o$ denote the related quantity that can be observed. For example, $o$ could be the noisy microphone time domain signals and the corresponding mouth sequence, or parameters that describe relevant features of the mouth movement, e.g. open-mouth area, open-mouth height and width, etc., a time-frame of the noisy microphone signal and the corresponding mouth image, etc.

It is well-known that the minimum mean-square error (mmse) estimator of S given observation $o$ is given by the condition-mean estimator,

$$\hat{S} = E(S \mid o) = \int s f_{s|o}(s \mid o)\, do,$$

where $E(\bullet)$ is the statistical expectation operator, and where $f_{s|o}(s|o)$ is the (generally high-dimensional) conditional probability density (pdf) of the random variable $S$ given the observation $o$. This pdf may be estimated in an off-line calibration process, based on a large amount of $(s,o)$ observations, such as observations of noise-free speech signals $s$ and the corresponding noisy signals combined with related mouth information $o$. Given the statistical information about the observed and desired signals (i.e., the conditional pdf $f_{s|o}(s|o)$), other optimality criteria mmse are possible, e.g., maximum a posteriori, maximum likelihood, etc.

When a second hearing device including an image capture device is positioned at an opposite ear of the user, as illustrated in Fig. 4, the method may be expanded with a step of correlating images or image sequences from the two image capture devices to determine distance and/or angular direction to an object present in both images or image sequences from the two image capture devices. Two hearing devices, or at least the image capture device parts thereof, may be positioned at a distance between them, e.g. one at each ear, but as mentioned other positions are possible e.g. body-worn or the frame of a set of glasses. This allows for further enhanced detection possibilities based on image analysis, e.g. determining distance and/or angle to an object in the images, as the distance to the object will be reflected by the relation of the position of the object in each image and the distance between the two image capture devices.

Using the images from the image capture device, the processor analyse images from the image capture device to detect vertical and/or horizontal movement of the head of the user. This is then utilized in the directional microphone system for adapting directionality of the directional microphone system. The detected movements include changes in head orientation of the user, e.g. tilt and/or turn.

In a further use of the method, the images from the analysing image or image sequence are used for recognising a person as a talker known to the user, e.g. via a database of known talkers.

One or more characteristics of a person may be stored and used for recognising the person using the image capture device. This may include face features, such as eye, nose and /or mouth position each relative the each of the others or other suitable visual characteristic used for face detection. When a person is then recognised the hearing device processing may be adapted according to the recognized talker, e.g. if the person is a spouse certain processing may be desired, while other members of the users family may require other types of processing, e.g. speech enhancement specifically aimed at enhancing speech from children.

Fig. 8 is a schematic illustration of a hearing device positioned at the ear of the user. The hearing instrument comprises two image capture devices, one facing forward and one facing backwards. The two image capture devices may be operated simultaneously or independently. The two image capture devices are connected to the same processor, alternatively, each image capture device is connected to a respective processor.

Combinations of the above embodiments and many other embodiments will be apparent to those of skill in the art upon reading and understanding the above description.

The apparatus and/or method steps as set out in the claims may be implemented by means of hardware, software, firmware or any combination of these. Some of the features could also be implemented as software running on one or more data processors and/or digital signal processors.

The individual elements of any of the disclosed embodiments may be physically, functionally and logically implemented in any suitable way such as in a single unit, in a plurality of units or as part of separate functional units. It is intended that the structural features of the devices described above, in the detailed description and in the claims may be combined with steps of the method, when appropriately substituted by a corresponding process. Embodiments of the method have the same advantages as the corresponding systems.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

**Claims**

1. Hearing device having a first part configured to be positioned behind the pinna of a user and a second part configured to be positioned in the ear canal of the user, a third part configured to mechanically connect the first part to the second part, the first part comprising:

   an input transducer for receiving sound signal,
   a processor for processing the received sound signal,
   an image capture device in communication with the processor, the image capture device positioned in the housing so that the image capture captures images in the direction of the nose of the wearer, and
   the processor is configured to detect presence of a face via the image capture device, and determine time instants of voice presence and voice absence from the face, and the processor is adapted to operate signal processing algorithms based on the detection, and
   the hearing device including an output transducer for providing the processed sound signal perceivable as sound to the user, the output transducer being positioned in the first part or the second part.

2. The hearing device according to claim 1, wherein the determination of time instants of the voice presence and voice absence is based on a combination of the image capture device and the input transducer.

3. The hearing device according to any one of the claims 1-2, wherein the image capture device is positioned remote from a housing of the hearing device and the image capture device is in wired or wireless communication with the processor.

4. The hearing device according to any one of claims 1-3, wherein the processor is configured to detect lip movement in image sequences captured by the image capture device, such as the processor is configured to identifying presence of vowels and/or consonants in speech, and/or the processor is configured to determining words spoken via lip reading.

5. The hearing device according to claim 4, wherein the processor is further adapted to analyse the sound signal to detect speech envelope and correlate the speech envelope to detected lip movement to identify direction to an active speech source relative to the image capture device.

6. The hearing device according to any one of claims 1-5, wherein the input transducer includes a directional microphone system having adaptable directionality, and wherein the processor is configured to adapt the directionality of the directional microphone system based on a signal from the processor.

7. A binaural hearing system comprising a first and a second hearing device each comprising:

   a first part configured to be positioned behind the pinna of a user and a second part configured to be positioned in the ear canal of the user, a third part configured to mechanically connect the first part to the second part, the first part comprising:

   an input transducer for receiving sound signal, a processor for processing the received sound signal,
   an image capture device in communication with the processor, the image capture device positioned in the housing so that the image capture captures images in the direction of the nose of the wearer, and the processor is configured to detect presence of a face via the image capture device, and determine time instants of voice presence and voice absence from the face, and the processor is adapted to operate signal processing algorithms based on the detection, and the hearing device including an output transducer for providing the processed sound signal perceivable as sound to the user, the output transducer being positioned in the first part or the second part.

8. The binaural hearing system according to claim 7, wherein the first hearing device includes a communication device for communicating with the other hearing device positioned at an opposite ear of the user, and the second hearing device includes an image capture device, wherein the processor is configured to determine distance to an object recorded by both cameras and the processor is further configured to adapt directionality of the directional microphone system accordingly.

9. The binaural hearing system of claim 7 or 8, wherein the image capture device is a 3D camera.

10. A method of operating a hearing device including an input transducer for receiving sound signal, a processor for processing the received sound signal, an output transducer for providing the processed sound signal perceivable as sound to the user, and an image capture device in communication with the processor, the image capture device arranged behind the pinna of a user, the method comprising:

   the image capture device capturing one image or a sequence of images,
   the processor detects presence of a face via the image capture device and determine time instants of voice presence and voice absence from the face and
the processor adapting the processing of the sound signal accordingly.

11. The method according to claim 10, wherein the input transducer includes a directional microphone system having adaptable directionality, the method further comprising a step of detecting a face in the image or sequence of images and adapting the directionality accordingly.

12. The method according to claim 10 or 11, wherein the processor detects vowels and/or consonants in speech from the face, or the processor performs speech recognition.

13. The method according to any one of claims 10-12, wherein in the event that multiple faces are detected in the image or sequence of images, the method includes detecting lip movements of the detected faces, and the processor analyses the sound signal to detect speech envelope and correlate the speech envelope to detected lip movement to identify direction to an active speech source relative to the image capture device.

14. The method according to any one of claims 10-13, wherein when a second hearing device including an image capture device is positioned at an opposite ear of the user, the method includes correlating images or image sequences from the two cameras to determine distance to an object in both the images or image sequences.

15. The method according to any one of claims 10-14, wherein the processor analyse images from the image capture device to detect vertical and/or horizontal movement of the head of the user.

Fig. 1

Fig. 2

Fig. 3

Fig. 4

Fig. 5

Fig. 6

Fig. 7

Fig. 8

Europäisches
Patentamt
European
Patent Office
Office européen
des brevets

# EUROPEAN SEARCH REPORT

Application Number

EP 15 20 1019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/099370 A1 (MOORE KEITH E [US]) 29 May 2003 (2003-05-29) | 1,3,4,7, 9,10,12, 15 | INV. H04R25/00 A61B5/1171 |
| Y | * paragraph [0010] - paragraph [0021] * * paragraph [0027] - paragraph [0032] * * paragraph [0033] - paragraph [0036] * * figures 1,2,3 * | 2,5,6,8, 11,13,14 | G06K9/00 G10L25/78 G10L15/25 H04R3/00 |
| Y | US 2004/267521 A1 (CUTLER ROSS [US] ET AL) 30 December 2004 (2004-12-30) * paragraph [0005]; figure 1 * * paragraph [0008] - paragraph [0016] * * paragraph [0051] - paragraph [0056] * * paragraph [0064] - paragraph [0069] * * paragraph [0075] * | 2,5,13 | |
| Y | US 5 940 118 A (VAN SCHYNDEL ANDRE J [CA]) 17 August 1999 (1999-08-17) * column 4, line 20 - line 34 * * column 5, line 20 - column 6, line 17 * * column 7, line 35 - line 67 * * figures 1,2,4a,4b * | 6,8,11, 14 | |
| A | KR 101 250 934 B1 (CHUNG YOON SUK [KR]) 9 April 2013 (2013-04-09) * paragraphs [0040], [0047], [0049] * * paragraphs [0054], [0066] * * paragraph [0123] * | 3 | TECHNICAL FIELDS SEARCHED (IPC) H04R A61B G06K G10L |
| A | US 2006/104454 A1 (GUITARTE PEREZ JESUS F [DE] ET AL) 18 May 2006 (2006-05-18) * the whole document * | 1-15 | |

The present search report has been drawn up for all claims

| Place of search | Date of completion of the search | Examiner |
|---|---|---|
| The Hague | 19 May 2016 | Valenzuela, Miriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 20 1019

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2016

| Patent document cited in search report | | Publication date | Patent family member(s) | Publication date |
|---|---|---|---|---|
| US 2003099370 | A1 | 29-05-2003 | NONE | |
| US 2004267521 | A1 | 30-12-2004 | NONE | |
| US 5940118 | A | 17-08-1999 | NONE | |
| KR 101250934 | B1 | 09-04-2013 | NONE | |
| US 2006104454 | A1 | 18-05-2006 | DE 102004000043 A1<br>EP 1667113 A2<br>US 2006104454 A1 | 24-05-2006<br>07-06-2006<br>18-05-2006 |

EPO FORM P0459

For more details about this annex : see Official Journal of the European Patent Office, No. 12/82